Europäisches Patentamt

European Patent Office

Office européen des brevets

(11) Publication number: **0 327 156**
**A1**

## EUROPEAN PATENT APPLICATION

(21) Application number: 89200154.6

(22) Date of filing: 26.01.89

(51) Int. Cl.4: **C12P 41/00 , C12P 13/04 ,**
**//(C12P13/04,C12R1:38)**

(30) Priority: 29.01.88 NL 8800224

(43) Date of publication of application:
09.08.89 Bulletin 89/32

(84) Designated Contracting States:
AT BE CH DE ES FR GB GR IT LI NL SE

(71) Applicant: STAMICARBON B.V.
Mijnweg 1
NL-6167 AC Geleen(NL)

(72) Inventor: Kerkhoffs, Pieter Laurens
van Goghstraat 5
NL-6165 VG Geleen(NL)
Inventor: Van Balken, Johannes Arnoldus
Maria
Schrijnewerkerstraat 1
NL-6114 XH Susteren(NL)

(54) Process for the preparation of sulphur-containing L-alpha-amino acids.

(57) Process for the preparation of sulphur- containing L-alpha-amino acids consisting of contacting a sulphur-containing L- or DL-alpha-amino acid amide with an aminopeptidase obtained from a culture of Pseudomonas putida or related microorganisms, such as Pseudomonas reptilivora or Pseudomonas arvilla, and isolating the resultant sulphur-containing L-alpha-amino acid, the enzymatic reaction being carried out in the presence of a hydroxylamine compound.

EP 0 327 156 A1

# PROCESS FOR THE PREPARATION OF SULPHUR-CONTAINING L-ALPHA-AMINO ACIDS

The invention relates to a process for the preparation of sulphur-containing L-alpha-amino acids consisting of contacting a sulphur-containing L- or DL-alpha-amino acid amide with an aminopeptidase obtained from a culture of Pseudomonas putida or related microorganisms, such as Pseudomonas reptilivora or Pseudomonas arvilla, and isolating the resultant L-alpha-amino acid.

It is generally known that certain microorganisms, such as Aspergillus oryzae, Aspergillus parasiticus, Mycobacterium phlei, Aeromonas proteolytica, Bacillus subtilis and Bacillus stearothermophilus, can form enzymes that are capable of hydrolyzing alpha-amino acid amides in an aqueous medium, with formation of alpha-amino acid. It should be noted that where in this application reference is made to alpha-amino acid amides, this term is meant to include the salts derived from these amides, such as the corresponding sulphates, hydrochlorides, nitrates, acetates, etc. Enzyme preparations with an activity as indicated above can be obtained from animal organs, such as for instance cow's eyes or pig's kidneys.

These known enzymes, also referred to as alpha-aminopeptidases, show a highly stereospecific activity and strong preference for the hydrolysis of L-alpha-amino acid amides. The D-alpha amino acid amides are consequently hardly affected by these enzymes.

These alpha-aminopeptidases are therefore suitable for effecting an optical separation of DL-alpha amino acids. In such a process, alpha-aminopeptidase is contacted with DL-alpha-amino acid amide in order to effect a hydrolysis of the L-alpha-amino acid amide with formation of the corresponding amino acid, after which the resultant amino acid and/or the remaining D-alpha-amino acid amide is isolated (compare Greenstein & Winitz: "Chemistry of the aminoacids", vol.3, pp.1778-1781, New York, 1961).

The aminopeptidase can be used in this process in its pure form or as a raw, aminopeptidase-containing preparation, and optionally also in immobilized form, for instance absorbed or chemically bound to a carrier.

In US-A-4.080.259 a process is described for the preparation of an L-alpha-amino acid (for instance L-phenyl glycine) and a D-alpha-amino acid amide (such as D-phenyl glycinamide) from a DL-alpha-amino acid amide by contacting the DL-alpha-amino acid amide with an aminopeptidase preparation which has been obtained from Pseudomonas putida, Pseudomonas reptilovora or Pseudomonas arvilla. This aminopeptidase activity has also been demonstrated for other alpha-amino acid amides, including sulphur-containing alpha-amino acid amides. The yield calculations in said patent specification are based on analysis of the amino acid content and therefore give no absolute indications about the stereoselectivity of the reaction for all alpha-amino acids.

Is has been found now that when applying this process to sulphur-containing L- or DL-alpha-amino acid amides, for instance of methionine, homomethionine, thienyglycine, cysteine and cystine, no optically pure alpha-amino acids present virtually completely in the L-form are obtained. Research has shown that during the process considerable racemization of the formed L-alpha-amino acid occurs. The D-amino acid amide remains unaffected, however. As a consequence it is difficult to isolate these alpha-amino acids in the L-form. The racemization occurring under the process conditions is attributed to the fact that the aminopeptidase preparation, which has been obtained for instance from a culture of Pseudomonas putida, also contains a racemase for sulphur-containing amino acids. The object of the invention now is to suppress this racemization fully or virtually fully.

It has been found now that the racemase activity can be inhibited by taking up in the reaction mixture a hydroxylamine compound, by which are understood hydroxylamine or O-substituted hydroxylamine or salts of these compounds.

The process according to the invention for the preparation of sulphur-containing L-alpha-amino acids by contacting a sulphur-containing L- or DL-alpha-amino acid amide with an aminopeptidase obtained from a culture of Pseudomonas putida or related microorganisms, such as Pseudomonas reptilivora or Pseudomonas arvilla, and isolating the formed sulphur-containing L-alpha-amino acid, is characterized in that the enzymatic reaction is carried out in the presence of a hydroxylamine compound.

The object of the invention is thus accomplished in a simple manner, which has not been utilized until now.

By cultivating said microorganisms in a manner known in itself, preparations are obtained with a particularly high peptidase activity, but also with considerable racemase activity with respect to sulphur-containing amino acids.

The microorganisms used in the process according to the invention are described in Bergey's Manual of determinative bacteriology (Baltimore 1975).

Stocks that are used by preference are Pseudomonas putida ATCC 12633, ATCC 25571, ATCC 17390, ATCC 17426 and ATCC 17484, Pseudomonas reptilovora ATCC 14039 and Pseudomonas arvilla ATCC 23974.

These stocks can be obtained from American Type Culture Collection, at Washington D.C, USA.

Pseudomonas putida, in particular stock ATCC 12633, is a microorganism that is particularly preferable.

The microorganisms used in accordance with the invention can be cultivated in usual nutrient media, such as described for instance by Hegeman in Journal of Bacteriology, 91, page 1140 (1966).

The microorganisms are preferably cultivated at a temperature within the range of 30-35¤C under aerobic conditions.

In most cases addition of growth factors or inductors is not required. Addition of yeast extract has a favourable effect on the production of the enzyme. After an incubation period of about 2 to 30 hours the cells can be collected, preferably during the period of exponential growth.

A preparation with high alpha-aminopeptidase activity can be obtained by precipitation of the cells, optionally with application of a flocculation agent. The cells may also be entangled or bonded to or absorbed to a carrier.

In some cases it may be desirable to modify the cell walls, for instance by a thermal treatment or by making the enzyme better accessible. A raw preparation can also be obtained by destroying the cells and recovering the enzyme by extraction, filtration and optionally drying by nebulizing.

A preparation consisting of the pure enzyme can be obtained in the usual manner from the raw product described above. Pure enzyme or enzyme preparations can also be obtained by generally known techniques from the culture medium.

A suitable preparation can be obtained by cultivating a microorganism belonging to the Pseudomonas putida, Pseudomonas reptilivora and Pseudomonas arvilla in the presence of a nutrient medium containing assimilable sources of carbon, nitrogen and phosphor.

In a suitable embodiment of the process according to the invention, a quantity of hydroxylamine compound is added to the alpha-amino acid amide before the preparation with alpha-aminopeptidase activity is contacted with the sulphur-containing L- or DL-alpha-amino acid amide. The amount of hydroxylamine compound is chosen on the basis of the quantity of enzyme preparation. As hydroxylamine compound can be used hydroxylamine or O-substituted hydroxylamine, but salts of it can also be used, for instance Na-, K-, NH₄-, Mg-, Ca- salts. Examples of O-substituted hydroxylamines are: O-methyl, O-ethyl, O-propyl, O-isopropyl, O-chloroallyl-, hydroxylamine.

The addition of the hydroxylamine compound can of course also be effected directly to the enzyme preparation before the latter is contacted with the sulphur-containing L- or DL-alpha-amino acid amide. Simultaneous supply, whether or not via a joint supplying device, of the enzyme preparation and the hydroxylamine compound to the sulphur-containing L- or DL-alpha-amino acid amide (or inversely) is also possible, as well as in-situ formation of the hydroxylamine compound. If the supply of the hydroxylamine compound is effected after the contacting of the enzyme preparation and the sulphur-containing L- or DL- alpha amino acid amide, occurrence of some racemization of the formed L-alpha amino acid is inevitable. This effect is greater as the addition of the hydroxylamine compound takes place later.

The contact between the preparation with alpha-aminopeptidase activity and the sulphur-containing L- or DL-alpha-amino acid amide in the presence of the hydroxylamine compound preferably takes place in an aqueous medium at a temperature between 0 and 60¤C. A temperature between 20 and 40¤C is preferred in particular. The pH should be between 6 and 12, preferably between 7.5 and 10.5.

Outside said ranges the activity and/or the stability of the enzyme is/are in general insufficient for use in practice. The enzyme can be activated in a manner known in itself, for instance by addition of a metal compound, such as a magnesium, manganese or zinc compound.

The weight ratio between the (unpurified) enzyme and the substrate may vary within wide limits, for instance between 1 : 25 and 1 : 750. If a pure enzyme is applied, a higher ratio can be used.

The quantity of hydroxylamine compound which is used according to the invention is easily determined by one skilled in the art, for instance in relation with the activity of the enzyme preparation, and as a rule amounts to 0.5-5 wt.% relative to the quantity of enzyme preparation. Preferably a quantity of 0.8 to 2.0 wt.% per part by weight of enzyme preparation is used.

When the hydrolysis of the sulphur-containing L-alpha-amino acid amide is completed, the free acid can be separated from the remaining D-alpha-amino acid amide. The latter compound can be hydrolized, with the D-alpha-amino acid being formed.

The process according to the invention is therefore suitable for isolation of optically pure, natural or synthetic, sulphur-containing L-alpha-amino acids, such as methionine, homomethionine, thienylglycine, cysteine or cystine.

The invention will now be described in detail with reference to the following examples, which only serve to elucidate the invention and do not restrict it.

Comparative example A

In a 50 ml three-neck flask, provided with stirrer, cooler and heating, 32.45 g (0.156 mole) DL-methionine amide acetate was dissolved in 202.5 g water at room temperature. With 50% KOH the solution was brought to pH = 9.0 and then heated to 40°C. Subsequently, 5.9 g of an enzyme preparation of Pseudomonas putida (ATCC 12633) was added and the reaction mixture was kept at 40°C for 24 hours with stirring. After cooling to room temperature the enzyme was subsequently separated off by centrifugation, after which benzaldehyde was added to the solution at pH = 9.0. The resulting precipitate, which analysis showed to consist of the Schiff base of benzaldehyde with D-methionine amide, was filtered off. The filtrate was then acidified to pH = 4.5 with hydrochloric acid, which again resulted in a precipitate. Filtration of this precipitate yielded 11.0 g methionine (0.07 mole). Analysis by means of a polarimeter showed that the methionine obtained was 56.5% in the L-form.
$[alpha]_D^{23} = + 0.250$ (c = 8; 6 n HCl).

Example I

The above test of comparative example A was repeated, but prior to addition of the enzyme preparation, 0.050 g hydroxylamine (about 1 wt.% relative to the enzyme preparation) was added to the solution. Again, a methionine yield of 11.0 g (0.07 mole) was obtained. Analysis showed that more than 99.2% of this product was obtained in the L-form.
$[alpha]_D^{23} = + 23.62$ (c = 8; 6 n HCl).

Comparative example B

The test described in comparative example A was repeated, but instead of DL-methionine amide acetate, 44.0 g DL-2-thienylglycine amide acetate (0.2 mole) was started from. The yield of amino acid after processing was 12.6 g (0.08 mole) 2-thienylglycine, of which 55% in the L-form.
$[alpha]_D^{23} = + 7.5$ (c = 1; H₂O).

Example II

The experiment of comparative example B was repeated, but prior to the addition of the enzyme preparation, 75 mg hydroxylamine (about 1.5 wt.% relative to the enzyme preparation) was now added to the solution. The yield of 2-thienylglycine after processing was 12.0 g (0.076 mole), with a selectivity of ≥99.5% to the L-form.
$[alpha]_D^{23} = + 74.0$ (c = 1; H₂O).

## Claims

1. Process for the preparation of sulphur-containing L-alpha-amino acids consisting of contacting a sulphur-containing L-or DL-alpha-amino acid amide with an aminopeptidase obtained from a culture of Pseudomonas putida or related microorganisms, such as Pseudomonas reptilivora or Pseudomonas arvilla, and isolating the resultant sulphur-containing L-alpha-amino acid, characterized in that the enzymatic reaction is carried out in the presence of a hydroxylamine compound.

2. Process according to claim 1, characterized in that the enzymatic reaction takes place in an aqueous medium.

3. Process according to claim 1 or 2, characterized in that the enzymatic reaction takes place at a temperature between 0 and 60°C.

4. Process according to any one of the claims 1-3, characterized in that the enzymatic reaction takes place between 20 and 40°C.

5. Process according to any one of the claims 1-4, characterized in that the enzymatic reaction takes place at a pH between 6 and 12.

6. Process according to any one of the claims 1-5, characterized in that the enzymatic reaction takes place at a pH between 7.5 and 10.5.

7. Process according to any one of the claims 1-6, characterized in that the quantity of hydroxylamine compound amounts to 0.5 to 5 wt.% relative to the quantity of enzyme preparation.

8. Process according to claim 7, characterized in that the quantity of hydroxylamine compound amounts to 0.8 to 2 wt.% relative to the quantity of enzyme preparation.

9. Process according to any one of the claims 1-8, characterized in that hydroxylamine is used as hydroxylamine compound.

10. Process for the preparation of sulphur-containing L-alpha-amino acids through contact of a sulphur-containing L- or DL-alpha-amino acid amide with an aminopeptidase from a culture of Pseudomonas putida or related microorganisms such as substantially described in the description and/or the examples.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US-A-4 080 259 (W.H. BOESTEN)<br>* Claims * | 1 | C 12 P 41/00<br>C 12 P 13/04 //<br>(C 12 P 13/04<br>C 12 R 1:38 ) |

TECHNICAL FIELDS
SEARCHED (Int. Cl.4)

C 12 P

The present search report has been drawn up for all claims

| Place of search | Date of completion of the search | Examiner |
|---|---|---|
| THE HAGUE | 02-05-1989 | DELANGHE L.L.M. |

EPO FORM 1503 03.82 (P0401)